# EUROPÄISCHE PATENTSCHRIFT

(11) **EP 0 432 364 B1**
(45) Veröffentlichungstag und Bekanntmachung des Hinweises auf die Patenterteilung: **17.02.1993**
(21) Anmeldenummer: 90115885.7
(22) Anmeldetag: 20.08.1990
(51) Int. Cl.: B01J 8/02, B01D 53/02, C01B 3/12, C01C 1/04, C07C 1/04, C07C 5/02, C07C 29/15

(54) **Reaktor zur Durchführung katalytischer Gasreaktionen bzw. physikalischer Trennoperationen**
Reactor for carrying out catalytic gas reactions or physical separation processes
Réacteurs pour exécuter des réactions catalytiques en phase gazeuse ou des procédés de séparation physique

(30) Priorität: 30.11.1989 DE 3939544; 13.06.1990 DE 9006659 U
(43) Veröffentlichungstag der Anmeldung: 19.06.1991
(73) Patentinhaber: Uhde GmbH, 44141 Dortmund (DE)
(72) Erfinder: Bähnisch, Hans-Joachim, D-4600 Dortmund 76 (DE)
(74) Vertreter: Patentanwälte Meinke, Dabringhaus und Partner

(56) Entgegenhaltungen:
- DE-C- 969 635
- DE-U- 9 000 604
- IT-A- 852 935
- PATENT ABSTRACTS OF JAPAN, Band 7, Nr. 167 (C-177)[1312], 22. Juli 1983, Seite 141 C 177;
- Chemical & Engineering 8, 1962, S. 60/61

## Beschreibung

Die Erfindung betrifft einen Reaktor zur Durchführung katalytischer Gasreaktionen bzw. physikalischer Trennoperationen mit einem im wesentlichen kugelförmigen, druckfesten Reaktormantel und einem im Reaktor angeordneten kugelförmigen Katalysatorbett sowie mit einem Synthesegaseintritt mit einem im Zentrum des kugelförmigen Katalysatorbettes angeordneten, im wesentlichen kugelförmigen Synthesegasverteiler mit einer perforierten Außenoberfläche und mit Synthesegasaustritten an der Peripherie des Katalysatorbettes zur Bildung einer definierten Strömung des Synthesegases im wesentlichen radial von innen nach außen durch das Katalysatorbett hindurch.

Aus einer Zeitschrift "Chemical & Engineering", Oktober 8, 1962, Seiten 60/61, ist eine gattungsgemäße Reaktorkonstruktion bekannt, wobei dort ein vergleichsweise stark dimensioniertes, in das Innere des Reaktorbettes weisendes Zuführ- bzw. Abzugsrohr mit einem perforierten sphärischen Verteiler bzw. Sammlerkopf offenbart ist. Der Katalysator wird dort in einem im wesentlichen kugelförmigen Katalysatortragnetz untergebracht, welches mittels Rippen von der Reaktorwand auf Abstand gehalten wird, um so einen Spalt zu bilden, durch den das Gas einem Abzugsstutzen zugeführt werden kann. Eine besondere Einflußnahme auf die Strömung durch das Katalysatorbett durch konstruktive Maßnahmen ist dort nicht angegeben.

Aus der italienischen Patentanmeldung IT 852 935 ist ein Reaktor bekannt, welcher einen äußeren druckdichten, etwa kugelförmigen Reaktormantel aufweist, in welchem zwischen einer inneren und einer äußeren kugelförmigen, mit Öffnungen versehenen Katalysatorummantelung ein kugelförmiges Katalysatorbett angeordnet ist. Im Seitenbereich des äußeren Reaktormantels ist ein horizontaler Synthesegaseintritt angeordnet, der durch das Katalysatorbett hindurch in das Innere der inneren Katalysatorummantelung in weitere Verteilrohre mündet, die ihrerseits in den zwischen der äußeren Katalysatorummantelung und dem Reaktormantel gebildeten kugelschalenförmigen Raum münden. Weiterhin ist an der gegenüberliegenden Seite des Synthesegaseintritts am Reaktormantel ein vom Innenraum der inneren Katalysatorummantelung ausgehender horizontaler Synthesegasaustritt angeordnet. Das Synthesegas wird über den Synthesegaseintritt und die Verteileinrichtungen in den kugelschalenförmigen Raum geführt und strömt von dort etwa radial von außen nach innen durch das Katalysatorbett, in welchem die Reaktion stattfindet, um anschließend in den Innenraum der inneren Katalysatorummantelung einzutreten und durch den Synthesegasaustritt abgeführt zu werden.

Die bekannten kugelförmigen Reaktoren zeichnen sich im Vergleich zu sonst üblichen zylinderförmigen Reaktoren insbesondere durch eine wesentlich höhere Druckfestigkeit aus, d.h. es können bei vergleichbaren Reaktormantelmaterialien geringere Wandstärken verwendet oder bei etwa gleichen Wandstärken können einfachere, weniger feste Mantelmaterialien eingesetzt werden, wodurch die Herstellungskosten entsprechend wesentlich verringert werden. Von Nachteil bei der Lösung nach der IT 852 935 ist jedoch der relativ aufwendige und komplizierte Reaktoraufbau, da zur Ausbildung des kugelschalenförmigen Synthesegasverteilraumes zusätzlich zum Reaktormantel eine äußere Katalysatorummantelung und außerdem aufwendige Gasverteileinrichtungen zur Leitung des Synthesegases vom Zentrum in den kugelschalenförmigen Raum notwendig sind. Vor allem hat sich herausgestellt, daß sich im Katalysatorbett kein definiertes, vorherbestimmbares Strömungsprofil einstellt, so daß es schwierig ist, die Synthesereaktion im gewünschten Maße durchführen zu können.

Aufgabe der Erfindung ist die Schaffung einer Lösung, mit der ein derartiger kugelförmiger Reaktor vereinfacht und insbesondere ein definiertes Strömungsprofil im Katalysatorbett eingestellt werden kann.

Mit einem Reaktor der eingangs bezeichneten Art wird diese Aufgabe erfindungsgemäß dadurch gelöst, daß im Inneren des Synthesegasverteilers zur Strömungsleitung eine Innenkugel angeordnet und als Synthesegasaustritte eine Mehrzahl am Reaktormantel verteilt angeordnete Gasaustritte mit den Reaktormantel durchdringenden Gassammelleitungen gebildet ist.

Durch diese Ausbildung wird der Reaktor vereinfacht, da keine aufwendigen Verteileinrichtungen für das Synthesegas und neben dem Reaktormantel keine das Katalysatorbett begrenzenden weiteren Katalysatorummantelungen erforderlich sind. Durch die erfindungsgemäße Ausbildung des Synthesegasverteilers und des Synthesegasaustritts mit der Mehrzahl von Gasaustritten wird im Katalysatorbett eine weitgehend genau definierte Strömung des Synthesegases radial von innen nach außen erreicht, wobei der Reaktionsraum nach außen hin zunimmt, was aufgrund des nach außen hin zunehmenden Reaktionsablaufes und der ebenfalls zunehmenden Wärmeentwicklung bei exothermen Reaktionen von entsprechend großem Vorteil ist.

Aus der DE-PS 969 635 ist ein kleiner, in einen Ofen einzubauender Gasbrenner mit Zündvorrichtung bekannt mit einem zentrischen, innenliegenden, kugelförmigen Hohlkörper.

Die DE-36 13 903-A1 zeigt zum katalytischen Dehydrieren einen zylindrischen Reaktor mit Gaszuführung von unten, wobei die DE-OS 24 24 664 einen kugelförmigen Reaktor zur Ammoniak-und Methanolsynthese zeigt, in dem ein schwebendes Katalysatorbett angeordnet ist.

In vorteilhafter Ausgestaltung der Erfindung ist vorgesehen, daß die Gasaustritte jeweils einen mit dem Katalysatorbett in Verbindung stehenden Gassammelkörper mit perforierter Kontaktfläche aufweisen, wobei der Gassammelkörper bevorzugt konisch, korbförmig, zylinderförmig oder scheibenförmig ausgebildet ist. Durch diese Ausgestaltungen kann eine besonders große Austrittsfläche am jeweiligen Gassammelkörper bereitgestellt werden, wodurch entsprechend ein definiertes Strömungsprofil des Synthesegases im wesentlichen radial von innen nach außen gewährleistet wird und Totzonen im Katalysatorbett vermieden werden.

Vorteilhaft ist es, wenn die Gassammelleitungen der Gasaustritte außerhalb des Reaktormantels mit einer zentralen Gasabführleitung verbunden sind. Diese Gasabführleitung ist dann außerhalb des Reaktormantels angeordnet.

In weiterer Ausgestaltung sieht die Erfindung vorteilhaft auch vor, daß im Bereich der Reaktoroberseite ein gefüllter Katalysatordom zum Volumenausgleich beim erstmaligen Anfahren des Reaktors angeordnet ist. Bei Verwendung spezieller Katalysatoren, z.B. von Kupfer-Katalysatoren, die in der Anfahrphase des Reaktors um etwa 10 % schrumpfen, wird dann auf konstruktiv besonders einfache Weise ein selbsttätiges Nachrutschen von Katalysator und damit eine vollständige Befüllung des Reaktorinnenraumes mit Katalysator gewährleistet.

Konstruktiv besonders günstig ist es dann, daß der Synthesegaseintritt durch den Katalysatordom geführt ist. Es ist dann nicht notwendig, am kugelförmigen Reaktoraußenmantel einen zusätzlichen Anschlußflansch vorzusehen, vielmehr kann der Anschlußflansch des Katalysatordoms entsprechend auch für den Synthesegaseintritt benutzt werden.

Eine weitere Ausgestaltung der Erfindung sieht vor, daß der Synthesegasverteiler zusätzlich mit einer Quenchgaszuführung verbunden ist, wodurch eine Temperaturregelung, insbesondere Kühlung, im Reaktor möglich ist. Es kann aber auch ein dem Reaktor nachgeschalteter Wärmeaustauscher zum Wärmeaustausch außerhalb des Reaktors vorgesehen sein.

Der erfindungsgemäße Reaktor ist insbesondere zur Verwendung zur Methanol- oder Ammoniaksynthese, zur CO-Konvertierung, zur Methanisierung oder zur Hydrierung bzw. selektiven Hydrierung von Kohlenwasserstoffen oder zur Aufnahme von physikalisch arbeitenden Massen geeignet, wie Aktivkohle oder Zinkoxid, z.B. zur Adsorption von Schwefelwasserstoff, Kohlendioxid und Quecksilber.

Zur Optimierung der Gasführung sieht die Erfindung in Ausgestaltung vor, daß im Inneren des Synthesegasverteilers zur Strömungsleitung eine Innenkugel angeordnet ist, wobei vorteilhaft die Innenkugel an Haltestegen im Inneren des Synthesegasverteilers angeordnet ist, damit ein vorbestimmter Strömungsquerschnitt im Inneren des Gasverteilers definiert ist.

Zur besseren Anpassung an das sich setzende Katalysatormaterial sieht die Erfindung dabei in Ausgestaltung vor, daß der Synthesegasverteiler an einem im wesentlichen zentrischen Gaszuführungsrohr angeordnet ist, welches mit einer Längenkompensationsstrecke ausgerüstet ist.

Dieses Merkmal der Erfindung trägt dem Gedanken Rechnung, daß nach einer gewissen Betriebsdauer (Reduktion des Katalysators) nach Neufüllung des Reaktors mit Katalysatormaterial dieses sich um ein gewisses Maß verdichtet und damit setzt, so daß evtl. Hohl- oder Fehlstellen im "Schatten" des zentrischen Synthesegasverteilers durch das Setzen des Reaktormateriales entstehen könnten. Bei den hohen Strömungeschwindigkeiten würde dies zur Zerstörung des Granulates führen und damit zum Zusetzen des Katalysators, dem hilft die Längenkompensationsstrecke ab, die für ein optimales Mitführen des Synthesegasverteilers sorgt.

Ausgestaltungen bestehen dabei nach der Erfindung darin, daß das Gaszuführungsrohr in Schwerkraftrichtung von oben an dem Synthesegasverteiler angeordnet ist und eine Längenkompensation im Lagerbereich des Reaktormantels vorgesehen ist und/oder daß die Kompensationsstrecke von einem elastischen Ausgleichselement mit dessen Festigkeit erhöhenden Stützelementen ausgebildet ist.

Zum optimalen Ableiten am Reaktormantel sieht die Erfindung auch vor, daß an der Innenfläche des Reaktormantels, wie an sich bekannt, eine Mehrzahl von Gasaustritten vorgesehen sind, wobei die Gasaustritte als Sammelkörper spinnennetzartige Sammelrohre aufweisen.

Eine andere Art der Synthesegasableitung besteht erfindungsgemäß darin, daß die Gassammelrohre an der Innenfläche des Reaktormantels als der Reaktormantelkrümmung angepaßte, einen Kreisausschnitt darstellende Sammelrohre ausgebildet sind.

Die Erfindung ist nachstehend anhand der Zeichnung beispielsweise näher erlauert. Diese zeigt in
Fig. 1 einen vereinfachten Schnitt durch einen Reaktor,
Fig. 2 ein abgewandeltes Ausführungsbeispiel in gleicher Darstellung wie Fig. 1 sowie in
Fig. 3 ebenfalls einen Ausschnitt aus einem vereinfacht wiedergegebenen Reaktor nach einem weiteren Ausführungsbeispiel der Erfindung.
Fig. 4 ein Verfahrensfließbild einer Methanolsynthese unter Einsatz erfindungsgemäßer Reaktoren nach Fig. 1.

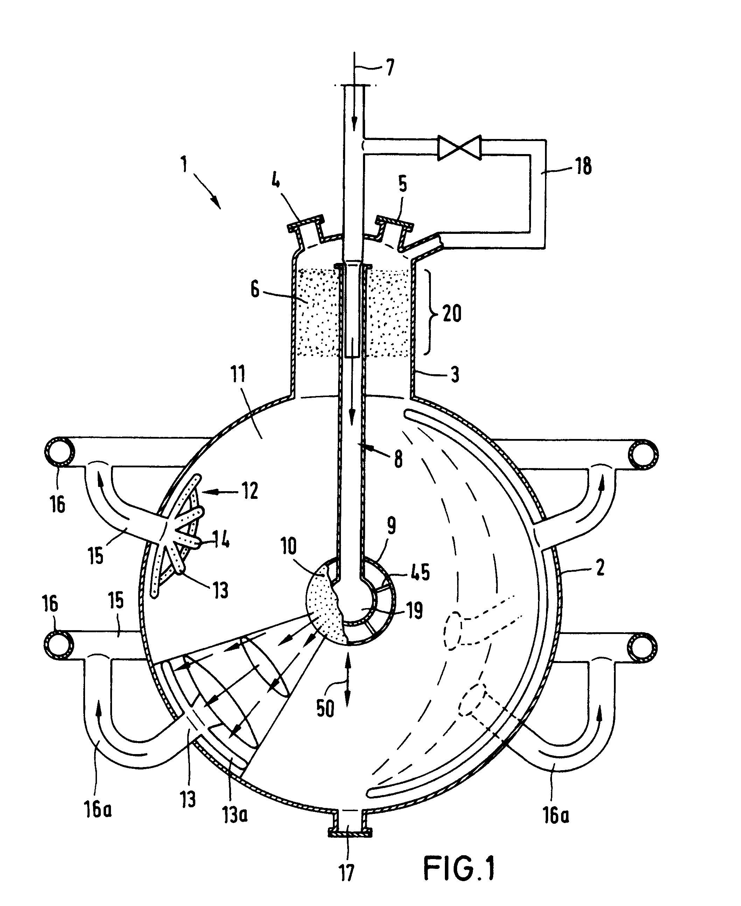

Ein Reaktor zur Durchführung katalytischer Gasreaktionen, insbesondere für die Methanol- oder Ammoniaksynthese, die Entschwefelung, die CO-Konvertierung, die Methanisierung oder die selektive Hydrierung, ist in der Fig. 1 allgemein mit 1 bezeichnet. Der Reaktor 1 weist zunächst einen im wesentlichen kugelförmigen, druckfesten Reaktormantel 2 auf, an dessen Oberseite ein Katalysatordom 3 druckdicht angeordnet ist. Der Katalysatordom 3 ist mit einem Katalysatorfüllstutzen 4 und einem Mannloch 5 versehen und enthält ein mit 6 bezeichnetes Katalysatorvorhaltevolumen.

An der Oberseite des Katalysatordomes 3 und damit ebenfalls an der Oberseite des Reaktors ist ein Synthesegaseintritt angeordnet, der in Fig. 1 durch den Pfeil 7 angedeutet ist. Dieser Gaseintritt 7 weist eine Leitung 8 auf, die durch den Katalysatordom 3 geführt und in einem im Zentrum des kugelförmigen Reaktormantels 2 angeordneten kugelförmigen Synthesegasverteiler 9 mündet. Der Synthesegasverteiler 9 weist eine perforierte Außenfläche 10 auf und ist im Zentrum des mit 11 bezeichneten kugelförmigen Katalysatorbetts im Reaktormantel 1 angeordnet.

Der Synthesegasaustritt ist von einer Mehrzahl von Gasaustritten 12 gebildet, die bevorzugt symmetrisch verteilt an der Innenseite des Reaktormantels 2 angeordnet sind. Die Gasaustritte 12 weisen jeweils einen spinnennetzartigen bzw. strahlenförmigen Gassammelkörper 13 mit perforierter Kontaktfläche 14 innerhalb des Reaktors auf, wobei die Sammelkörper 13 eine unterschiedliche Raumform aufweisen können. In Fig. 1 sind zusätzlich in der linken Figurenhälfte rohrbogenförmige Sammelkörper 13a dargestellt.

Selbstverständlich ist bei einem Reaktor für alle Gassammelkörper 13 nur eine einheitliche Raumform vorgesehen, um ein einheitliches Strömungsprofil im Reaktor zu gewährleisten. Jeder Gassammelkörper 13 bzw. 13a ist mit einer Gassammelleitung 15 versehen, die den Reaktormantel 2 durchdringt und in Gasabführleitungen 16 außerhalb des Reaktormantels 2 mündet. Diese Gasabführleitungen 16 sind vorzugsweise in nicht näher dargestellter zentraler Gasabführleitung zusammengeführt; die Strömungsrichtung ist durch Pfeile 16a angedeutet.

An der Reaktorunterseite ist ein Katalysatorauslaßstutzen 17 angeordnet, durch den verbrauchter Katalysator entnommen werden kann. Am Katalysatordom 3 kann zusätzlich auch noch eine Reduktionseinrichtung für den Katalysator vorgesehen sein, die in der Zeichnung nur durch eine Anschlußleitung 18 angedeutet ist, die vom Synthesegaseintritt 7 abzweigt und in den Katalysatordom 3 mündet.

Die Besonderheit der in Fig. 1 dargestellten Konstruktion besteht in zwei Dingen: Zum einen ist im Inneren des Synthesegasverteilers 9 eine Strömungsleitkugel 19 vorgesehen, zum anderen weist das Gaszuführungsrohr 8 eine Längenkompensationsstrecke, allgemein mit 20 bezeichnet, auf, wobei diese Kombinationsstrecke durch Ineinandertauchen der Rohrstutzenbereiche ausgebildet ist.

Die Innenkugel 19 als Strömungsleitkugel ist im Ausführungsbeispiel gemäß Fig. 1 über Haltestege 45 ortsfest gelagert.

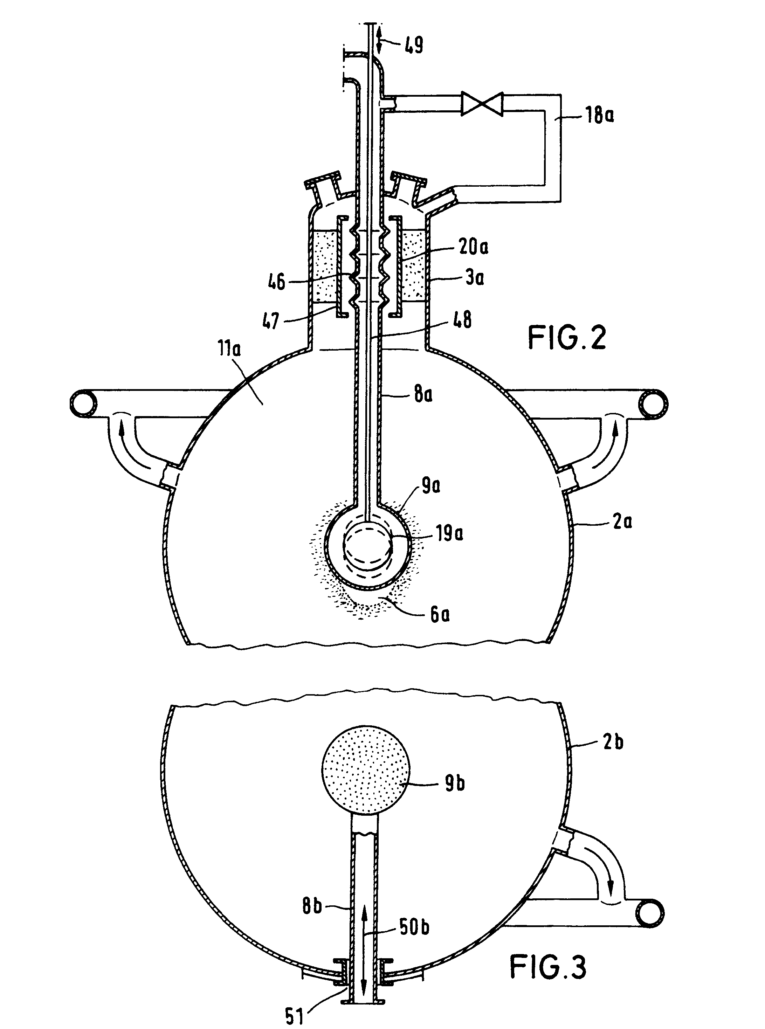

In Fig. 2 ist ein abgewandeltes Ausführungsbeispiel dargestellt, dort wird die Längenkompensationsstrecke 20a und einem in Längsrichtung verformbaren Rohrbereich 46 gebildet mit äußerem Stützrohr 47. Der Sinn einer derartigen Längenkompensationsstrecke 20 bzw. 20a besteht im wesentlichen darin, nach Neufüllung des Reaktors mit Granulat und dessen Verdichtung während des Betriebes (z.B. Reduktion), eine Art Schattenbildung hinter dem Synthesegasverteiler 9a zu verhindern, in Fig. 2 ist dieser mit 6a bezeichnet. Hier kann durch die Längenkompensationsstrecke 20a die Synthesegasverteilkugeln nachsacken.

Um ggf. die im Inneren des Synthesegasverteilers 9a befindliche Innenkugel 19a in ihrer Lage verändern zu können, kann vorgesehen sein, die Innenkugel 19a an einer Steuerstange 48 anzuordnen. Die Steuerbewegung ist in Fig. 2 oben mit dem Doppelpfeil 49 angedeutet, der Pfeil der das Nachrutschen des Synthesegasverteilers 9a darstellt, ist in Fig. 1 als Doppelpfeil mit 50 bezeichnet.

In Fig. 3 ist schließlich eine andere Darstellung der Anordnung der Innenkugel 9b wiedergegeben, welche mit der Ansteuerung von unten, das Gaszuführungsrohr ist dort mit 8b bezeichnet, die Nachführungsmöglichkeit mit dem Doppelpfeil 50b. Die Längenkompensation ist dort mit 51 bezeichnet.

In Fig. 4 ist die Anwendung eines Reaktors nach Fig. 1 dargestellt, wobei Fig. 4 eine Methanolsyntheseanlage zeigt, in der drei kugelförmige Reaktoren 1 vorgesehen sind.

Frischgas 21 und Kreislaufgas 22 werden zunächst zu einem Gasstrom 23 gemischt. Dieser Gasstrom 23 wird anschließend in einem Verdichter 24 zu einem Gasstrom 25 verdichtet. Dieser verdichtete Gasstrom 25 wird dann mit einem CO₂-Gasstrom 26 zu einem Gasstrom 27 vermischt.

Der Gasstrom 27 wird daraufhin in einem Gas-/Gaswärmeaustauscher 28 bzw. in der Anfahrphase in einem Anfahrwärmeaustauscher 29, der mit einem externen Heizmedium 30 beaufschlagt ist, vorgewärmt. Der vorgewärmte Synthesegasstrom 27 wird dann in den ersten kugelförmigen Reaktor 1 eingeleitet, wobei nach Reaktion in demselben das austretende Gas über die einzelnen Gasababführleitungen 16 in eine zentrale Gasabführleitung 16′ geleitet wird. Das Methanolproduktgas der Leitung 16′ wird dann in einem ersten Mitteldruck-Dampferzeuger 31, dessen Mitteldruckdampfleitung mit 32 angedeutet ist, abgekühlt und anschließend wird das abgekühlte Methanolproduktgas 33 in einen zweiten kugelförmigen Reaktor 1 eingeleitet.

Nach Durchströmung des zweiten Kugelreaktors 1 und Reaktion in demselben erfolgt eine weitere Abkühlung des entstandenen Methanolproduktgases 34 in einem zweiten Mitteldruck-Dampferzeuger 31a, in dem Mitteldruckdampf 32a erzeugt wird.

Das abgekühlte Methanol-Produktgas 34 wird dann ggf. weiteren Reaktoren 1 zugeführt, je nach den gewünschten Reaktionsabläufen, letztendlich gelangt es jedoch in einen letzten kugelförmigen Reaktor 1, dessen Produktgas 35 dann zur Abkühlung durch den Gas-/Gaswärmeaustauscher 28 geleitet wird. Das abgekühlte Produktgas 36 kann dann in einem Luftkühler 37 und ggf. in einem Kondensator 38 weiter abgekühlt werden, dessen Kühlwasserleitung mit 39 bezeichnet ist.

Das abgekühlte Produktgas 40 wird dann in einem vorzugsweise zweigeteilten Abscheider 41 in Rohmethanol 42, Kreislaufgas 22, Entspannungsgas 43 und Purgegas 44 getrennt.

Natürlich ist die Erfindung nicht auf die in der Zeichnung dargestellten Ausführungsbeispiele beschränkt. Weitere Ausgestaltungen der Erfindung sind möglich, ohne den Grundgedanken zu verlassen. So kann der erfindungsgemäße Reaktor selbstverständlich auch für andere katalytische Gasreaktionen und insbesondere auch für physikalische Trennoperationen eingesetzt werden.

## Patentansprüche

1. Reaktor zur Durchführung katalytischer Gasreaktionen bzw. physikalischer Trennoperationen mit einem im wesentlichen kugelförmigen, druckfesten Reaktormantel und einem im Reaktor angeordneten kugelförmigen Katalysatorbett sowie mit einem Synthesegaseintritt mit einem im Zentrum des kugelförmigen Katalysatorbettes angeordneten, im wesentlichen kugelförmigen Synthesegasverteiler mit einer perforierten Außenoberfläche und mit Synthesegasaustritten an der Peripherie des Katalysatorbettes zur Bildung einer definierten Strömung des Synthesegases im wesentlichen radial von Innen nach Außen durch das Katalysatorbett hindurch,
dadurch gekennzeichnet,
daß im Inneren des Synthesegasverteilers (9) zur Strömungsleitung eine Innenkugel (19) angeordnet und als Synthesegasaustritte eine Mehrzahl am Reaktormantel (2) verteilt angeordnete Gasaustritte (12) mit den Reaktormantel (2) durchdringenden Gassammelleitungen (15) gebildet ist.

2. Reaktor nach Anspruch 1,
dadurch gekennzeichnet,
daß die Gasaustritte (12) jeweils einen mit dem Katalysatorbett (11) in Verbindung stehenden Gassammelkörper (13) mit perforierter Kontaktfläche (14) aufweisen.

3. Reaktor nach Anspruch 2,
dadurch gekennzeichnet,
daß die Gassammelleitungen (15) der Gasaustritte (12) außerhalb des Reaktormantels (2) mit einer zentralen Gasabführleitung (16′) verbunden sind.

4. Reaktor nach Anspruch 1 oder einem der folgenden,
dadurch gekennzeichnet,
daß im Bereich der Reaktoroberseite ein mit Katalysator gefüllter Katalysatordom (3) zum Volumenausgleich beim erstmaligen Anfahren des Reaktors angeordnet ist.

5. Reaktor nach einem der vorangehenden Ansprüche,
dadurch gekennzeichnet,
daß zur Einstellung der Lage der inneren Strömungsleitkugel eine das Gaszuführungsrohr (8) durchsetzende Steuerstange (48) vorgesehen ist.

6. Reaktor nach einem der vorangehenden Ansprüche,
dadurch gekennzeichnet,
daß der Synthesegasverteiler (9) an einem im wesentlichen zentrischen Gaszuführungsrohr (8) angeordnet ist, welches mit einer Längenkompensationsstrecke (20) ausgerüstet ist.

7. Reaktor nach einem der vorangehenden Ansprüche,
dadurch gekennzeichnet,
daß die Kompensationsstrecke von einem elastischen Ausgleichselement (46) mit dessen Festigkeit erhöhenden Stützelementen (47) ausgebildet ist.

8. Reaktor nach einem der vorangehenden Ansprüche,
dadurch gekennzeichnet,
daß an der Innenfläche des Reaktormantels (2), wie an sich bekannt, eine Mehrzahl von Gasaustritten (12) vorgesehen sind, wobei die Gasaustritte als Sammelkörper spinnennetzartige Sammelrohre (13) aufweisen.

9. Reaktor nach Anspruch 8,
dadurch gekennzeichnet,
daß die Gassammelrohre an der Innenfläche des Reaktormantels (2) als der Reaktormantelkrümmung angepaßte, einen Kreisausschnitt darstellende Sammelrohre (13a) ausgebildet sind.

10. Reaktor nach einem der vorangehenden Ansprüche,
dadurch gekennzeichnet,
daß der Synthesegaseintritt (7,8) durch den Katalysatordom (3) geführt ist.

11. Reaktor nach einem der vorangehenden Ansprüche,
dadurch gekennzeichnet,
daß das Gaszuführungsrohr (8b) in Schwerkraftsrichtung von unten an dem Synthesegasverteiler (9b) angeordnet ist und eine Längenkompensation (51) im Lagerbereich des Reaktormantels (2b) vorgesehen ist.

12. Reaktor nach einem der vorangehenden Ansprüche,
dadurch gekennzeichnet,
daß der Synthesegasverteiler (9,9a,9b) zusätzlich mit einer Quenchgaszuführung verbunden ist.

13. Verwendung eines Reaktors nach Anspruch 1 oder einem der folgenden zur Methanol- oder Ammoniaksynthese, zur CO-Konvertierung, zur Methanisierung oder zur Hydrierung bzw. selektiven Hydrierung von Kohlenwasserstoffen und/oder für die Adsorption mit Aktivkohle, Zinkoxid od. dgl.

## Claims

1. A reactor for carrying out catalytic gas reactions or physical separation operations comprising a substantially spherical pressure-resistant reactor casing and a spherical catalyst bed arranged in the reactor, and a synthesis gas intake with a substantially spherical synthesis gas distributor arranged in the centre of the spherical catalyst bed, with a perforated outside surface, and with synthesis gas outlets at the periphery of the catalyst bed for forming a defined flow for the synthesis gas substantially radially from the inside outwardly through the catalyst bed, characterised in that an inner sphere (19) is arranged in the interior of the synthesis gas distributor (9) for guiding the flow and a plurality of gas outlets (12) arranged in distributed array at the reactor casing (2), with gas collector manifolds (15) which pass through the reactor casing (2) is formed as the synthesis gas outlets.

2. A reactor according to claim 1 characterised in that the gas outlets (12) each have a respective gas collector body (13) which is communicated with the catalyst bed (11) and which has a perforated contact surface (14).

3. A reactor according to claim 2 characterised in that the gas collector manifolds (15) of the gas outlets (12) are connected outside the reactor casing (2) to a central gas discharge conduit (16′).

4. A reactor according to claim 1 or one of the following claims characterised in that disposed in the region of the top side of the reactor is a catalyst dome (3) which is filled with catalyst, for volume compensation purposes when the reactor is first started.

5. A reactor according to one of the preceding claims characterised in that a control rod (46) which passes through the gas feed pipe (8) is provided for adjusting the position of the inner flow-guidance sphere.

6. A reactor according to one of the preceding claims characterised in that the synthesis gas distributor (9) is arranged on a substantially central gas feed pipe (8) which is provided with a length compensation section (20).

7. A reactor according to one of the preceding claims characterised in that the compensation section is formed by a resilient compensating element (46) with support elements (47) for increasing the strength of the latter.

8. A reactor according to one of the preceding claims characterised in that a plurality of gas outlets (12) are provided at the inside surface of the reactor casing (2), as is known per se, the gas outlets having cobweb-like collector pipes (13) as collector bodies.

9. A reactor according to claim 8 characterised in that the gas collector pipes at the inside surface of the reactor casing (2) are in the form of collector pipes (13a) which are adapted to the reactor casing curvature and which represent a portion of a circle.

10. A reactor according to one of the preceding claims characterised in that the synthesis gas intake (7, 8) is passed through the catalyst dome (3).

11. A reactor according to one of the preceding claims characterised in that the gas feed pipe (8b) is arranged on the synthesis gas distributor (9b) from below in the direction of the force of gravity and a length compensation means (51) is provided in the mounting region of the reactor casing (2b).

12. A reactor according to one of the preceding claims characterised in that the synthesis gas distributor (9, 9a, 9b) is additionally connected to a quench gas feed.

13. Use of a reactor according to claim 1 or one of the following claims for the synthesis of methanol or ammonia, for CO-conversion, for methanisation or for the hydrogenation or selective hydrogenation of hydrocarbons and/or for adsorption with activated carbon, zinc oxide or the like.

## Revendications

1. Réacteur destiné à la réalisation de réactions gazeuses catalytiques ou d'opérations de séparation physique comportant une enveloppe de réacteur résistant à la pression, essentiellement sphérique et un lit de catalyseur sphérique agencé dans l'enveloppe du réacteur ainsi qu'une sortie de gaz de synthèse avec un distributeur de gaz de synthèse agencé au centre du lit de catalyseur sphérique, dans un distributeur de gaz de synthèse sensiblement sphérique avec une surface extérieure perforée et avec des sorties de gaz de synthèse sur la périphérie du lit de catalyseur pour la formation d'un écoulement défini de gaz de synthèse essentiellement radialement à partir de l'intérieur vers l'extérieur par le dit de catalyseur,
caractérisé en ce qu'à l'intérieur du distributeur de gaz de synthèse (9) est agencée une sphère interne (19) pour le guidage du courant et qu'en tant que sorties de gaz de synthèse est formée une pluralité de sorties de gaz (12) agencées sur l'enveloppe du réacteur (2) avec les conduites collectrices de gaz (15) traversant l'enveloppe de réacteur (2).

2. Réacteur selon la revendication 1,
caractérisé en ce que les sorties de gaz (12) comportent chaque fois un collecteur de gaz (13) avec une surface de contact perforée (14) en communication avec le lit de catalyseur (11).

3. Réacteur selon la revendication 2,
caractérisé en ce que les conduites collectrices de gaz (15) des sorties de gaz à l'extérieur de l'enveloppe du réacteur communiquent avec une conduite d'évacuation de gaz centrale (16′).

4. Réacteur selon la revendication 1 ou l'une des revendications suivantes, caractérisé en ce que dans la zone du côté supérieur du réacteur il est prévu un dôme de catalyseur (3) rempli de catalyseur pour la compensation de volume lors de la première mise en route du réacteur.

5. Réacteur selon l'une des revendications précédentes, caractérisé en ce que pour le réglage de la position de la sphère de guidage de courant interne il est prévu une tige de commande (48) traversant le tube de guidage de gaz (8).

6. Réacteur selon l'une des revendications précédentes caractérisé en ce que le distributeur de gaz de synthèse (9) est agencé sur un tube d'amenée de gaz (8) sensiblement central, lequel est muni d'un tronçon de compensation de longueur (20).

7. Réacteur selon l'une des revendications précédentes, caractérisé en ce que le tronçon de compensation de longueur est constitué par un élément compensatoire élastique (46) avec des éléments d'appui (47) augmentant sa résistance.

8. Réacteur selon l'une des revendications précédentes, caractérisé en ce que sur la surface intérieure de l'enveloppe du réacteur (2) comme cela est connu, sont prévues plusieurs de gaz (12), les sorties de gaz se présentant comme un ensemble collector de tubes collecteurs (13) en forme de toile d'araignée.

9. Réacteur selon la revendication 9, caractérisé en ce que les tubes collecteurs de gaz sur la surface interne de l'enveloppe de réacteur (2) sont deux tubes collecteurs (13a) avec une section circulaire adaptée à la courbure de l'enveloppe du réacteur.

10. Réacteur selon l'une des revendications précédentes, caractérisé en ce que l'entrée du gaz de synthèse (7,8) est menée à travers le dôme du catalyseur (3).

11. Réacteur selon l'une des revendications précédentes, caractérisé en ce que le tube d'amenée de gaz (8b) est agencé dans la direction de la force centrifuge à partir du bas sur le distributeur de gaz de synthèse (9) et il est muni d'un tronçon compensatoire de longueur (51) dans la zone de stockage de l'enveloppe du réacteur (2b).

12. Réacteur selon l'une des revendications précédentes, caractérisé en ce que le distributeur de gaz de synthèse (9, 9a, 9b) est de plus relié à une amenée de gaz d'extinction.

13. Utilisation d'un réacteur selon la revendication 1 ou l'une des revendications suivantes pour la synthèse du méthanol ou de l'amoniac pour la conversion CO, pour la métallisation ou pour l'hydrogénation, en l'occurence pour l'hydrogénation sélective d'hydrocarbures et/ou l'adsorption avec des charbons actifs, de l'oxyde de zinc ou des éléments analogues.
